# EUROPEAN PATENT SPECIFICATION

(11) **EP 3 752 233 B1**
(45) Date of publication and mention of the grant of the patent: **16.08.2023**
(21) Application number: 19754912.4
(22) Date of filing: 11.02.2019
(51) Int. Cl.: A61M 25/00, A61J 15/00

(54) **TUBE ASSEMBLY AND DISSOLVABLE TIP**
SCHLAUCHANORDNUNG UND LÖSLICHE SPITZE
ENSEMBLE TUBE ET POINTE SOLUBLE

(30) Priority: 13.02.2018 US 201862629989 P
(43) Date of publication of application: 23.12.2020
(60) Divisional application: 22192608.2
(73) Proprietor: Werd, LLC, Lexington, KY 40515 (US)
(72) Inventor: KIRN, David, S., Lexington, KY 40515 (US); HISEL, Richard, D., Nicholasville, KY 40356 (US); WHITMAN, William, Nicholasville, KY 40356 (US)
(74) Representative: Murgitroyd & Company
(86) International application number: PCT/US2019/017418
(87) International publication number: WO 2019/160785

(56) References cited:
- WO-A1-91/07200
- WO-A1-2017/007829
- GB-A- 2 337 464
- JP-A- 2013 116 220
- US-A- 2 603 217
- US-A- 5 049 138
- US-A- 5 281 212
- US-A1- 2002 198 440
- US-A1- 2010 049 165

## Description

### FIELD OF THE INVENTION

This document relates generally to temporary tubes for placement in a patient, and more specifically with tips used therewith.

### BACKGROUND OF THE INVENTION

In the course of healthcare, temporary tubes are placed into the body. Such tubes placed internally within the body are generally supplied with a tip, sometimes referred to as a bolus, at least in part to facilitate the insertion process. Tips facilitate insertion by having an arcuate shape along at least a portion of an outer surface. Generally, the outer surface may have a rounded or bullet shaped distal portion which enhances gliding and bending along curved, fragile surfaces of the nasal airway, the pharynx, the esophagus, stomach and/or intestines for example.

Accommodating the need to facilitate the insertion process, however, can result in a design compromise. After insertion, a tip designed to facilitate insertion could prevent unobstructed flow of material(s) passing out of the end of the tube. A partial or complete change away from axial material or fluid flow is required to exit via a pathway around or through the tip. Due to this necessary change of direction, the tip may interfere with such flow and thus contribute to complications associated with the tube, for example, clogging. Accordingly, a need exists for a tip designed to facilitate the insertion process and to limit any contribution to complications associated with the tube. Document WO2017007829 discloses a tube for placement within a patient, the tube having a lumen increasing in diameter along at least a portion of the tube to a distal end.

### SUMMARY OF THE INVENTION

In accordance with the purposes and benefits described herein, a tube assembly is provided. The tube assembly may be broadly described as including a tube for placement within a patient. The tube includes a lumen, a proximal end portion, and a distal end portion attached to a dissolvable tip. The invention is defined by the appended claims.

In another possible embodiment, the tip is partially inserted into the lumen within the distal end portion.

In still another possible embodiment, the tip includes a proximal end portion for insertion into the tube is less pliable than the tube. In another, the proximal end portion of the tip includes at least one of a ridge or a ring.

In yet another possible embodiment, the tip includes a central aperture. In still another, the tip is solid.

In one other possible embodiment, the tip includes an outer coating. In another, the outer coating is a water activated lubricious coating.

In yet still another possible embodiment, the tip includes at least one vent.

In another possible embodiment, an outer diameter of the tube and an outer diameter of the tip are substantially the same.

In still another possible embodiment, the tip includes a conical or bullet-shaped distal end.

In yet still another possible embodiment, the tube includes at least one vent.

In yet another possible embodiment, the tip includes at least one vent and the tube includes at least one corresponding vent. In another, the tip includes a collar that extends through the tube vent.

In another possible example, a tube assembly includes a tube for placement within a patient and a tip attached to a distal end portion of the tube. The tip is formed from a material dissolvable in a fluid.

In still another possible example, an enteral tube for placement within a patient includes a lumen and a distal end portion having an orifice therein, and a tip inserted into the lumen at a distal end of the tube, the tip having an angled face for directed matter out of the orifice.

In another example, a tip for use with a tube for placement within a patient includes a distal end for insertion into the patient, and a proximal end for engaging the tube wherein at least the proximal end comprises a soluble material.

In another possible example, an of the tip comprises a soluble material.

In still another possible example, the soluble material is any material or combination of materials which are soluble and suitable for human or animal consumption.

In yet another possible example, the soluble material is a polymer.

In the following description, there are shown and described several preferred embodiments of temporary tubes. As it should be realized, the tubes are capable of other, different embodiments and their several details are capable of modification in various, obvious aspects all without departing from the tubes and systems as set forth and described in the following claims. Accordingly, the drawings and descriptions should be regarded as illustrative in nature and not as restrictive.

### BRIEF DESCRIPTION OF THE DRAWING FIGURES

The accompanying drawing figures incorporated herein and forming a part of the specification, illustrate several aspects of the invention and together with the description serve to explain certain principles thereof. In the drawing figures:
Figure 1 is a perspective view of an enteral tube illustrating a distal end portion of a tube and a tip;
Figure 2 is a perspective view of a tip;
Figure 3 is a perspective view of yet another embodiment of an enteral tube illustrating a tip encasing a distal end portion of a tube;
Figure 4 is another perspective view of the tip showing the proximal end thereof;
Figure 5a is a partial plan view of a tube having a gradually increasing lumen cross section in a portion positioned within a patient;
Figure 5b is a partial plan view of a tube having a gradually increasing lumen cross section and a uniform outer cross section;
Figure 6 is a perspective view of an alternate enteral tube illustrating a distal end portion of a tube and a tip;
Figure 7 is a side elevational view of another alternate tip showing an oblique connection between a tube and the tip; and
Figure 8 is a side elevational view of still another alternate tip showing a curved connection between a tube and the tip.

Reference will now be made in detail to the present described embodiments of the invention, examples of which are illustrated in the accompanying drawing figures, wherein like numerals are used to represent like elements.

### DETAILED DESCRIPTION

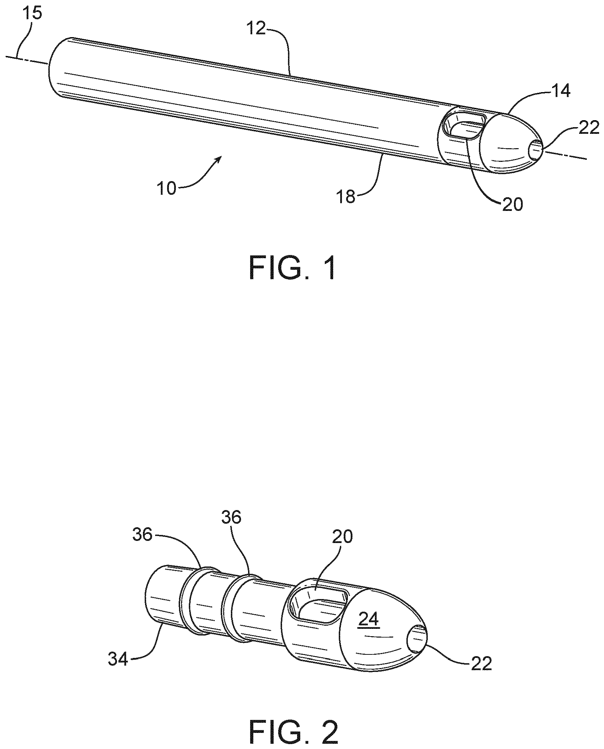

Reference is now made to Figure 1 which partially illustrates a tube 12 for placement within a patient (P). In this embodiment, the tube is an enteral tube which includes, but is not limited to, nasogastric tubes, nasoduodenal tubes, oral gastric tubes, oral duodenal tube, and feeding tubes located distal to the duodenum. Other embodiments may utilize other types of tubes, for example, gastrostomy or G-tubes inserted through the patient's abdomen to deliver nutrition directly to the stomach. While some designs utilize a bolster system designed to prevent withdrawal of the gastrostomy tube, others utilize other anchor means (e.g., inflatable balloons) to prevent withdrawal. In all designs, a dissolvable tip (solid or otherwise) may be utilized to improve the insertion process by limiting patient discomfort while ultimately allowing for the free flow of nutrients through the tube. One family of such gastrostomy devices is marketed under the brand MiniONE^{®} including balloon and non-balloon devices. As shown, a tube assembly 10 includes the enteral tube, or tube 12, and a tip 14.

The tube 12 includes a lumen extending a length of the tube from a proximal end portion (not shown) to a distal end portion 18 having an orifice therein. The described tip 14 defines a vent 20 and a distal aperture 22 for a guidewire (not shown) to pass through if desired. A square or butt connection between the tube 12 and tip 14 is shown with a proximal portion of the tip being hidden from view and positioned within the tube in this figure. As described below, other methods of attaching the tip 14 to the tube 12 may be utilized.

Since the tip 14 is only useful during tube insertion, the tip is made of a dissolvable material. In other words, the tip 14 is dissolvable. More specifically, the tip 14 is designed to remain intact to facilitate the insertion process and placement of the tube 12 and then dissolve away. This leaves an unobstructed distal opening in the tube 12 encompassing its full inner diameter and allowing fluid flow along the long axis of the tube (designated reference numeral 15 in Figure 1) without any change in direction. In other words, the tip 14 comprises a material, or materials, which are soluble and suitable for human or animal consumption. One soluble material is a polymer marketed under the trademark Affinisol^{®}. Broadly speaking, any soluble material may be utilized. In addition to being soluble, the material or materials may include additional properties such as lubriciousness when exposed to a moist environment. More specifically, the tip material may be coated with a water activated lubricious coating to facilitate gliding during insertion.

As best shown in Figure 2, the tip 14 includes a conical or bullet shaped distal contoured surface or face 24. In the described embodiment (best shown in Figure 1), an outer diameter of the tip 14 substantially corresponds to an outer diameter of the tube 12 to which it is attached creating a smooth outer surface even at the butt joint. Limiting the outer diameter of the tip 14 in these embodiments enhances patient comfort and eases the insertion process particularly when moving through narrower portions of the gastrointestinal tract.

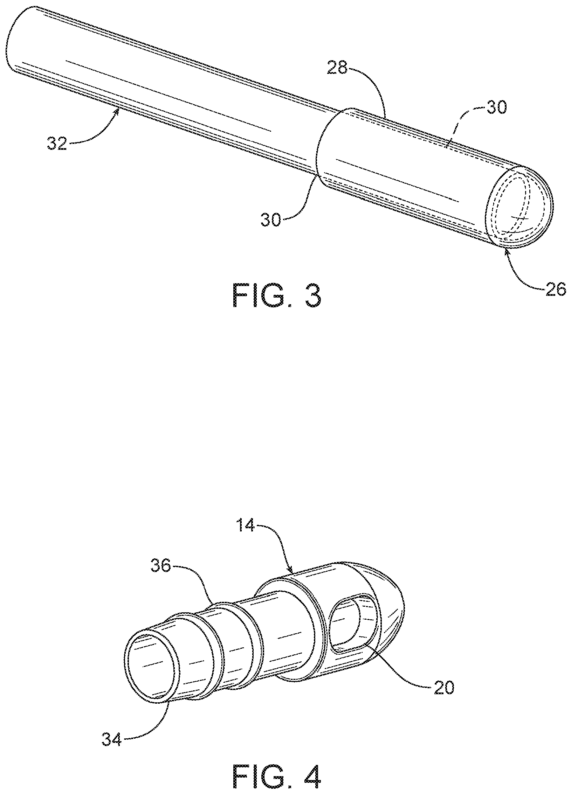

While maintaining the outer diameter of the tip to substantially correspond with the outer diameter of the tube provides certain advantages, other embodiments may include an outer tip diameter that is larger than the outer diameter of the tube. As shown in Figure 3, for example, an alternate tip 26 may include a proximal end portion 28 sized to receive a distal end portion 30 of a tube 32 therein. In this embodiment, the tip 26 encases or is attached to an outside diameter of the distal end portion 30 of the tube. In other words, the dissolving material may provide a covering over a distal end of the tube. The tip 26 may be formed by dipping the tube 32 in the dissolving material for example, may be pre-formed or molded, or otherwise.

As shown in Figure 4, the diameter of the described tip 14 is reduced to just less than an inner diameter of the tube 12 along a proximal portion 34 such that the tip 14 is held in place within the tube's distal end portion 18 (see Figure 1) by friction. Alternate embodiments, ensure retention of the tip 14 by means of adhesives, the natural tackiness of the tip material, or other retention means known in the art. One or more ridges 36, rings, or other mechanical features may be positioned on the proximal portion 34 of the tip 14 to provide retentive forces. Even more alternate embodiments may utilize a partially dissolvable tip. For example, the proximal portion 34 of the described tip 14 may be dissolvable to ensure the tip is dislocated from the tube 12 leaving an unobstructed distal opening in the tube 12 allowing fluid flow along the long axis of the tube. The remaining portion of the tip 14 may not be dissolvable but would dislodge from the tube 12 and pass through the gastrointestinal tract of the patient (P). In the embodiment shown in Figure 3, the portion of the tip 26 surrounding the distal end portion 30 of the tube 32 may be dissolvable to ensure the tip is dislocated from the tube while the remaining portion of the tip may not be dissolvable. In other embodiments, varying portions of the tip may be dissolvable so long as the dissolvable portions are sufficient to ensure the tip is dislocated from the tube.

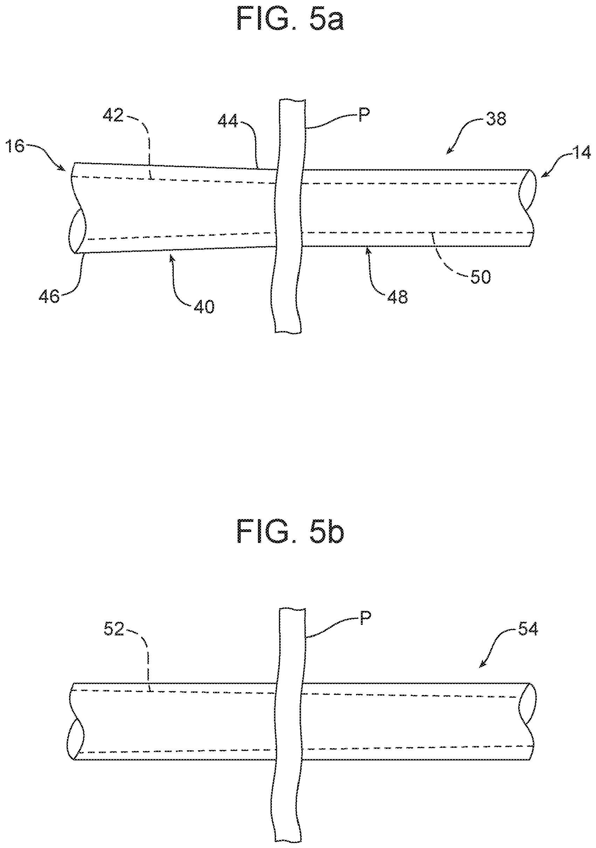

In alternate embodiments shown in Figures 5a and 5b, a tube 38 may have a tapered configuration. In other words, a portion 40 of the tube 38 which will reside within a patient (P) may have a gradually increasing lumen cross section 42 from a proximal end 44 to a distal end 46. A portion 48 of the tube 38 which remains external to the patient (P) may have a uniform lumen cross section 50 or, in an alternate embodiment, a tapered lumen cross section 52 may extend along an entirety of a tube 54, as shown in Figure 5b, or along a portion thereof. For example, the lumen cross section may gradually increase from a proximal end portion of tube 52 toward a distal end or a distal end portion of the tube 54. Even more, an outer cross section of the tube may be uniform, as shown in Figure 5b, or may gradually increase in areas where the lumen cross section gradually increases as shown in Figure 5a. If the tube is round, the lumen cross section will necessarily be an inner diameter of the tube.

The purpose of providing a gradually increasing lumen cross section is to provide a smaller cross section at the area which would be best for patient comfort, such as the portion of the tube 38 residing in the nose. Then, distal to this, having the gradual increase in cross section makes clogging less likely. When the tube 38 is in place, the patient (P) will not be able to detect the larger cross section of the tube distally.

Further, should a clog develop in the tapered portion 40 of the tube 38 within the patient (P), gentle pressure with a suitable fluid flush will make clearing the clog more likely than with a uniform lumen cross section. To further diminish the risk of clogging, an interior surface of the tube 38 may be coated in a non-stick or lubricious material. Clogs located in the constant lumen cross section portion 48 of the tube 38 would be mostly external to the patient (P). In this portion 48, external pressure may also be applied by the clinician to the tube 38 to milk or break up the clog and enhance clearing along with fluid flush.

In such alternate embodiments wherein the tube has a tapered inner diameter, the tip can be shaped to conform with the tapering inner diameter of the tube. For example, the proximal portion of the tip could be conically or frusto conically shaped.

Returning to the embodiment shown in Figure 1 and as suggested above, the tip 14 may define one or more apertures, holes, or vents 20. A central aperture or passageway 22 located axially along its length allows placement of the tube 12 by passing it over a guidewire (not shown) such is commonly utilized during fluoroscopic placement or endoscopic placement of feeding tubes. The central aperture 22 does not compromise placement with or without a stylet which remains completely within the lumen of the tube 12. The central aperture 22 offers two additional advantages. First, the central aperture 22 increases the surface area available for contact with fluids passing through the tube 12 and gastrointestinal fluids thereby expediting dissolution of the tip 14. Second, in the case of tube placement under fluoroscopic guidance, small amounts of contrast material may be passed through the tube 12 and central aperture 22 of the tip 14 facilitating confirmation of tube position. In alternate embodiments, the tip 14 may be solid or may have a central aperture with no additional holes or vents.

While a single vent 20 is shown throughout the figures, one or more vents may be integrated into a tip to allow a higher flow rate of water, radiographic contrast media, nutritional formula, or other fluids. Importantly, each vent creates a minimally restricted flow pathway for any fluids passing down the lumen of the tube 12. Hence, patients may receive nutritional formula as soon as the tube 12 has been placed into the gastrointestinal tract with no need to wait until the tip 14 has completely or partially dissolved.

While allowing a higher flow rate of fluids, the vent 20 is formed such that the fluids exit the tube 12 via the vent at a substantially ninety-degree angle relative to the long axis of the tip 14 and tube. This configuration minimizes the risk that a guidewire inadvertently exists through the vent 20 rather than the central guide wire aperture 22. Nonetheless, other embodiments may include vents within a tip that exit at angles greater than and/or less than 90 degrees.

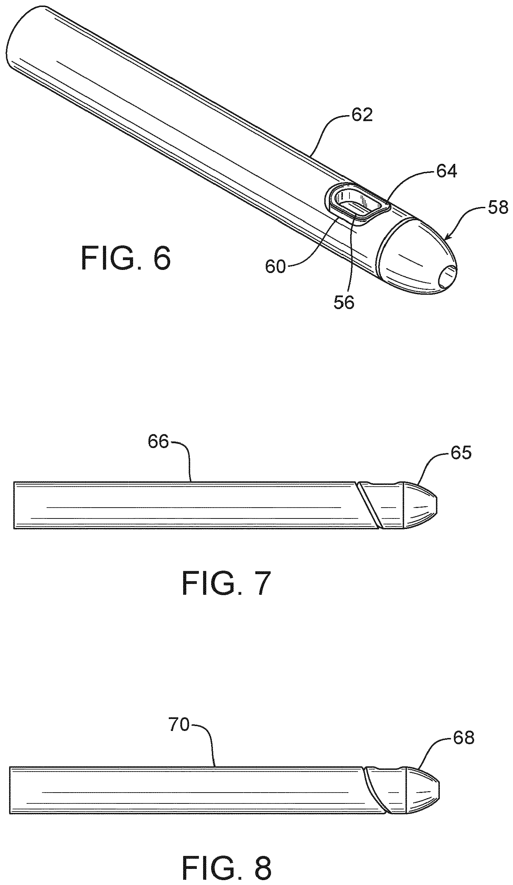

In the embodiment shown in Figure 1, the vent 20 obviates the need for a vent defined by the tube 12 with the benefit of lower costs. In alternate embodiments, however, the one or more tip vents may be positioned more proximally such that the vents open through a corresponding one or more vents defined by the tube. As shown in Figure 6, for example, a vent 56 formed in a tip 58 is located to cooperate with a vent 60 defined by a tube 62. In this arrangement, a cuff 64 extends through the tube vent 56 to provide further resistance preventing the tip 58 from coming out of the tube 62. In all embodiments, the vent(s) may include a uniform inside diameter or a nonuniform diameter such as a taper.

Following placement of the tube, the tip will reside in the gastrointestinal tract, typically, in the stomach (whether an enteral or gastrostomy tube) or small bowel. Both anatomic areas provide an aqueous environment which will lead to dissolution of the tip thereby leaving the end, or orifice, of the tube open and completely unobstructed. In addition, fluids such as water or nutritional formulas moving through the tip vent(s) and/or central aperture/hole, if one or both are present, further expedite dissolution of the tip. In an alternate embodiment of a solid dissolvable tip without a central hole or vents, the user may force the tip out of the tube by means of air or water pressure generated with a syringe placed at the proximal end of the tube. The ejected tip would then dissolve in the gastrointestinal tract in due course while immediately allowing fluids to move through the distal end of the tube.

As previously suggested, the tip 14 may be attached to the tube 12 utilizing a square or butt connection as shown in Figure 1. In addition, a tip 64 and tube 66 may be shaped to form an oblique connection therebetween as shown in Figure 7. Even more, a tip 68 and a tube 70 may be shaped to form a curved connection therebetween as shown in Figure 8.

The foregoing has been presented for purposes of illustration and description. It is not intended to be exhaustive or to limit the embodiments to the precise form disclosed. Obvious modifications and variations are possible in light of the above teachings. For example, one or more vents may be formed along the distal end portion 18 of the tube 12 shown in Figure 1. Such tube vents may be in place of vents formed in the tip 14 (e.g., vent 20) or may be in addition thereto. In addition, a radiopaque material such as barium, for example, may be incorporated in the tip to assist in location of the tip during fluoroscopic placement. Even more, the proximal aspect of the tip may be solid or have a lumen. The proximal internal edge may have a chamfered or rounded shape to facilitate passing of a guidewire from proximal to distal. Likewise, the proximal outside edge may also be chamfered or rounded to facilitate placement inside of a tube. Further, the outside diameter of the proximal portion may be tapered to facilitate placement inside of a tube. The distal shape of the lumen may be configured conically to further direct a guidewire through the central guidewire hole.

## Claims

1. An enteral tube assembly, comprising:
a tube (12) for placement within a patient, the tube having a lumen (42) increasing in diameter along at least a portion (40) of the tube to a distal end (46); and
a dissolvable tip (14) attached to the distal end wherein only a portion of the tip (14) is dissolvable to ensure that the tip is dislocated from the tube.

2. The tube assembly of claim 1 which is a nasogastric tube assembly.

3. The tube assembly of claim 1, wherein the dissolvable tip (14) is partially inserted into the tube.

4. The tube assembly of claim 3, wherein the dissolvable tip (14) includes a proximal end portion (34) for insertion into the tube (12), wherein the proximal end portion includes at least one ridge (36).

5. The tube assembly of claim 1, wherein the dissolvable tip (14) is partially inserted into the lumen (42).

6. The tube assembly of claim 5, wherein the dissolvable tip (14) includes a proximal end portion (34) for insertion into the lumen (42).

7. The tube assembly of claim 6, wherein the dissolvable tip (14) includes a central aperture (22).

8. The tube assembly of claim 6, wherein the dissolvable tip (14) includes at least one vent (20).

9. The tube assembly of claim 6, wherein an outer diameter of the tube (12) and an outer diameter of the tip (14) are substantially the same.

10. The tube assembly of claim 6, wherein the dissolvable tip (14) includes a conical distal end.

11. The tube assembly of claim 1, wherein the tube (22) includes at least one laterally facing vent (60).

12. The tube assembly of claim 11, wherein the dissolvable tip (14) includes a collar (64) extending through the tube vent (56).

13. The tube assembly of claim 1, wherein the dissolvable tip (58) includes at least one vent (56) and the tube (62) includes at least one corresponding vent (60).

14. The tube assembly of claim 13, wherein the dissolvable tip (58) includes a collar (64) extending at least partially through the tube vent (60).

15. The tube assembly of claim 1, further comprising a balloon.

## Patentansprüche

1. Eine enterale Schlauchanordnung, die Folgendes beinhaltet:
einen Schlauch (12) zum Platzieren innerhalb eines Patienten, wobei der Schlauch ein Lumen (42) aufweist, dessen Durchmesser sich entlang mindestens eines Abschnitts (40) des Schlauchs zu einem distalen Ende (46) erhöht; und
eine lösliche Spitze (14), die an dem distalen Ende angebracht ist, wobei nur ein Abschnitt der Spitze (14) löslich ist, um sicherzustellen, dass die Spitze von dem Schlauch abgelöst wird.

2. Schlauchanordnung gemäß Anspruch 1, die eine nasogastrische Schlauchanordnung ist.

3. Schlauchanordnung gemäß Anspruch 1, wobei die lösliche Spitze (14) teilweise in den Schlauch eingeführt wird.

4. Schlauchanordnung gemäß Anspruch 3, wobei die lösliche Spitze (14) einen proximalen Endabschnitt (34) zur Einführung in den Schlauch (12) umfasst, wobei der proximale Endabschnitt mindestens eine Rippe (36) umfasst.

5. Schlauchanordnung gemäß Anspruch 1, wobei die lösliche Spitze (14) teilweise in das Lumen (42) eingeführt wird.

6. Schlauchanordnung gemäß Anspruch 5, wobei die lösliche Spitze (14) einen proximalen Endabschnitt (34) zur Einführung in das Lumen (42) umfasst.

7. Schlauchanordnung gemäß Anspruch 6, wobei die lösliche Spitze (14) eine zentrale Öffnung (22) umfasst.

8. Schlauchanordnung gemäß Anspruch 6, wobei die lösliche Spitze (14) mindestens eine Abführung (20) umfasst.

9. Schlauchanordnung gemäß Anspruch 6, wobei ein Außendurchmesser des Schlauchs (12) und ein Außendurchmesser der Spitze (14) im Wesentlichen gleich sind.

10. Schlauchanordnung gemäß Anspruch 6, wobei die lösliche Spitze (14) ein konisches distales Ende umfasst.

11. Schlauchanordnung gemäß Anspruch 1, wobei der Schlauch (22) mindestens eine seitlich abgehende Abführung (60) umfasst.

12. Schlauchanordnung gemäß Anspruch 11, wobei die lösliche Spitze (14) einen Kragen (64) umfasst, der sich durch die Schlauchabführung (56) erstreckt.

13. Schlauchanordnung gemäß Anspruch 1, wobei die lösliche Spitze (58) mindestens eine Abführung (56) und der Schlauch (62) mindestens eine entsprechende Abführung (60) umfasst.

14. Schlauchanordnung gemäß Anspruch 13, wobei die lösliche Spitze (58) einen Kragen (64) umfasst, der sich mindestens teilweise durch die Schlauchabführung (60) erstreckt.

15. Schlauchanordnung gemäß Anspruch 1, die ferner einen Ballon beinhaltet.

## Revendications

1. Un ensemble formant sonde entérale, comprenant :
une sonde (12) destinée à être placée dans un patient, la sonde ayant une lumière (42) dont le diamètre augmente le long d'au moins une portion (40) de la sonde jusqu'à une extrémité distale (46) ; et
un embout (14) pouvant se dissoudre assujetti à l'extrémité distale, une portion seulement de l'embout (14) pouvant se dissoudre afin de garantir que l'embout soit disloqué de la sonde.

2. L'ensemble formant sonde de la revendication 1, lequel est un ensemble formant sonde nasogastrique.

3. L'ensemble formant sonde de la revendication 1, dans lequel l'embout (14) pouvant se dissoudre est inséré partiellement dans la sonde.

4. L'ensemble formant sonde de la revendication 3, dans lequel l'embout (14) pouvant se dissoudre inclut une portion d'extrémité proximale (34) destinée à être insérée dans la sonde (12), la portion d'extrémité proximale incluant au moins une nervure (36).

5. L'ensemble formant sonde de la revendication 1, dans lequel l'embout (14) pouvant se dissoudre est inséré partiellement dans la lumière (42).

6. L'ensemble formant sonde de la revendication 5, dans lequel l'embout (14) pouvant se dissoudre inclut une portion d'extrémité proximale (34) destinée à être insérée dans la lumière (42).

7. L'ensemble formant sonde de la revendication 6, dans lequel l'embout (14) pouvant se dissoudre inclut une ouverture centrale (22).

8. L'ensemble formant sonde de la revendication 6, dans lequel l'embout (14) pouvant se dissoudre inclut au moins un évent (20).

9. L'ensemble formant sonde de la revendication 6, dans lequel un diamètre externe de la sonde (12) et un diamètre externe de l'embout (14) sont substantiellement les mêmes.

10. L'ensemble formant sonde de la revendication 6, dans lequel l'embout (14) pouvant se dissoudre inclut une extrémité distale conique.

11. L'ensemble formant sonde de la revendication 1, dans lequel la sonde (22) inclut au moins un évent (60) orienté latéralement.

12. L'ensemble formant sonde de la revendication 11, dans lequel l'embout (14) pouvant se dissoudre inclut un collier (64) s'étendant à travers l'évent (56) de sonde.

13. L'ensemble formant sonde de la revendication 1, dans lequel l'embout pouvant se dissoudre (58) inclut au moins un évent (56) et la sonde (62) inclut au moins un évent (60) correspondant.

14. L'ensemble formant sonde de la revendication 13, dans lequel l'embout pouvant se dissoudre (58) inclut un collier (64) s'étendant au moins partiellement à travers l'évent (60) de sonde.

15. L'ensemble formant sonde de la revendication 1, comprenant en outre un ballonnet.
